(19) Europäisches Patentamt
European Patent Office
Office européen des brevets

(11) Veröffentlichungsnummer: **0 290 907 B1**

# (12) EUROPÄISCHE PATENTSCHRIFT

(45) Veröffentlichungstag der Patentschrift: **11.12.91**

(51) Int. Cl.5: **C07D 303/08**, C07D 303/22, C07D 303/34, C07D 407/06, C07D 233/60, C07D 249/08

(21) Anmeldenummer: **88107014.8**

(22) Anmeldetag: **02.05.88**

## (54) Verfahren zur Herstellung von Acetylenepoxiden und deren Verwendung.

(30) Priorität: **14.05.87 DE 3716022**

(43) Veröffentlichungstag der Anmeldung:
**17.11.88 Patentblatt 88/46**

(45) Bekanntmachung des Hinweises auf die Patenterteilung:
**11.12.91 Patentblatt 91/50**

(84) Benannte Vertragsstaaten:
**BE CH DE FR GB IT LI NL**

(56) Entgegenhaltungen:
**EP-A- 0 124 014**
**EP-A- 0 158 741**

**JOURNAL OF THE CHEMICAL SOCIETY, Sektion C, 1969, Seiten 12-15, London, GB; D. MILLER: "The synthesis of furans from acetylenic epoxides and diols"**

**EUROPEAN JOURNAL OF MEDICINAL CHEMISTRY-CHIMICA THERAPEUTICA, Band 19, Nr. 1, 1984, Seiten 23-27, Chatenay-Malabry, FR; H. GALONS et al.: "Synthèse et étude pharmacologique d'aryloxypropanolamines substituées sur le carbone 2"**

(73) Patentinhaber: **BAYER AG**
**W-5090 Leverkusen 1 Bayerwerk(DE)**

(72) Erfinder: **Lantzsch, Reinhard, Dr.**
**Am Buschhaeuschen 51**
**W-5600 Wuppertal 1(DE)**
Erfinder: **Schmetzer, Johannes, Dr.**
**Wacholderweg 45**
**W-5024 Pulheim-Sinthern(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein neues Verfahren zur Herstellung von Acetylenepoxiden und deren Verwendung als Zwischenprodukte zur Synthese von Stoffen mit fungizider Wirksamkeit.

Es sind bereits zahlreiche Acetylenepoxide bekannt (vgl. Compt. Rend. Acad. Sc. Paris, C 1968, 1085-1088; EP-A 0 052 424; EP-A 0 097 426 und EP-A 0 158 741). Verbindungen dieses Typs, in denen ein Kohlenstoffatom des Oxiran-Ringes sowohl mit der Acetylengruppe als auch mit einem substituierten Alkylrest verbunden ist, wurden bisher aber noch nicht offenbart.

Weiterhin wurden in der Literatur schon mehrere Verfahren zur Herstellung von Acetylenepoxiden beschrieben. So lassen sich derartige Substanzen durch metallorganische Reaktionen synthetisieren, indem man metallierte Acetylene mit Chlormethyl-ketonen umsetzt und die dabei entstehenden Chlormethyl-carbinole mit Basen behandelt (vgl. EP-A 0 052 424, EP-A 0 097 426 und EP-A 0 158 741). Diese Umsetzung kann durch das folgende Schema veranschaulicht werden:

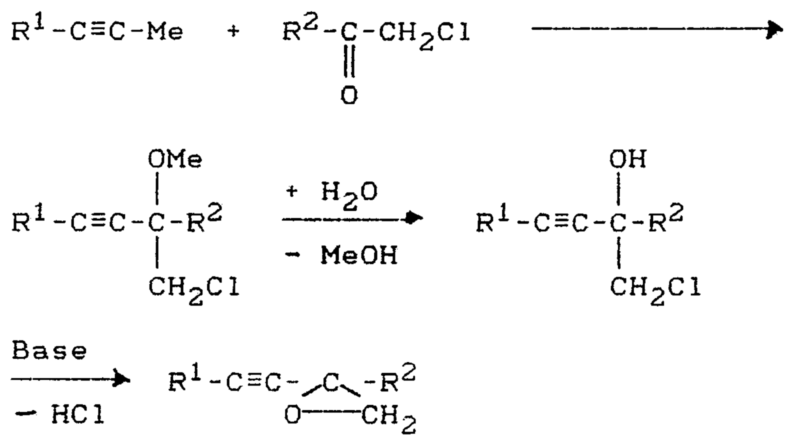


Me = Li, MgBr

Nachteilig an diesem Verfahren ist jedoch, daß metallorganische Reaktionen in technischem Maßstab nur problematisch durchführbar sind.

Wie ferner aus Compt. Rend. Acad. Sc. Paris, C 1968, 1085-1088 hervorgeht, sind bestimmte Acetylenepoxide dadurch zugänglich, daß die entsprechenden Acetylenketone mit Dimethylsulfonium-methylid umgesetzt werden. Die Ausbeuten an den gewünschten Produkten sind allerdings relativ gering. Ungünstig ist außerdem, daß sich die Ausbeuten bei längeren Reaktionszeiten durch Polymerenbildung vermindern.

Schließlich wird in der EP-A 0 052 424, der EP-A 0 097 426 und der EP-A 0 158 741 die Synthese von bestimmten Acetylenepoxiden durch Umsetzung von Acetylenketonen mit Trimethylsulfonium- oder Trimethyloxosulfonium-iodid vorgeschlagen. Es sind aber nur allgemeine Reaktionsgleichungen vorhanden. Beispiele oder genaue Angaben zu den erforderlichen Umsetzungsbedingungen sind nicht enthalten.

Es wurde nun gefunden, daß man Acetylenepoxide der Formel

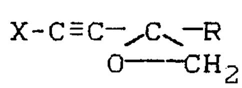

(I)

in welcher

X für gegebenenfalls substituiertes Phenyl oder Alkyl steht und

R für die Gruppierungen

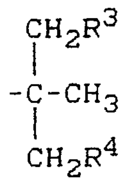 , 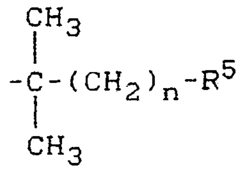 und 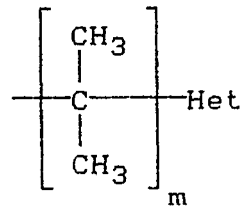

steht, wobei

- $R^3$ für Wasserstoff oder Halogen steht,
- $R^4$ für Halogen steht,
- $R^5$ für Alkylthio, sowie für jeweils gegebenenfalls substituiertes Phenyl, Phenoxy und Phenylthio, steht,
- n für die Zahlen 0, 1 oder 2 steht,
- m für die Zahlen 0 oder 1 steht und
- Het für gegebenenfalls substituiertes Dioxolanyl oder Dioxanyl steht,

erhält, wenn man Acetylenketone der Formel

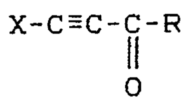 (II)

in welcher
R und X die oben angegebene Bedeutung haben,
mit Trimethylsulfoniumsalzen der Formel

$$(CH_3)_3\overset{\oplus}{S}\ \overset{\ominus}{Z} \qquad (III)$$

in welcher

- Z für Brom, Iod oder Methylsulfat steht,

in Gegenwart von Alkalimetall-tert.-butanolat und in Gegenwart von tert.-Butanol und gegebenenfalls Dimethylsulfid umsetzt.

Schließlich wurde gefunden, daß die Acetylenepoxide der Formel (I) sehr gut als Zwischenprodukte zur Synthese von bekannten Hydroxyalkinyl-azolyl-Derivaten mit fungizider Wirksamkeit geeignet sind.

Es ist als äußerst überraschend zu bezeichnen, daß die Acetylenepoxide der Formel (I) nach dem erfindungsgemäßen Verfahren in glatter Reaktion mit hohen Ausbeuten zugänglich sind. Aufgrund des bekannten Standes der Technik war nämlich zu erwarten, daß derartige Umsetzungen nur mit geringer Ausbeute ablaufen (vgl. Compt. Rend. Acad, Sc. Paris, C 1968, 1085-1088) oder gar nicht zu den gewünschten Produkten führen (vgl. J. Amer. Chem. Soc. 87, 4972 [1965] und J. Amer. Chem. Soc. 93, 2471 [1971]). Ferner war anzunehmen, daß die als Ausgangsverbindungen eingesetzten Acetylenketone unter den Reaktionsbedingungen zu einem beträchtlichen Anteil in Acetylene und Carbonsäure-Derivate gespalten werden (vgl. Houben-Weyl "Methoden der Organischen Chemie", Band V/2a, Georg Thieme Verlag, Stuttgart 1977, Seite 651). Außerdem war zu vermuten, daß die abgespaltenen Acetylene mit nicht umgesetzten Acetylenketonen weitere Umsetzungen eingehen würden. Überraschend ist schließlich auch, daß die erfindungsgemäße Umsetzung bei Verwendung anderer Lösungsmittel als tert.-Butanol nicht zu dem gewünschten Erfolg führt.

Das erfindungsgemäße Verfahren zeichnet sich durch eine Reihe von Vorteilen aus. So ermöglicht es die Herstellung von Acetylenepoxiden der Formel (I) in hohen Ausbeuten, wobei billige und gut zugängliche Verbindungen als Ausgangsprodukte eingesetzt werden. Ferner ist die Umsetzung einfach durchführbar und die Isolierung der Acetylenepoxide der Formel (I) bereitet keinerlei Schwierigkeiten. Das erfindungsgemäße Verfahren ist daher besonders geeignet, um Acetylenepoxide der Formel (I) in technischem Maßstab herzustellen.

Die erfindungsgemäß herstellbaren Acetylenepoxide der Formel (I) eröffnen einen neuen, vorteilhaften

Zugang zu den fungizid wirksamen Hydroxyalkinyl-azolyl-Derivaten der Formel

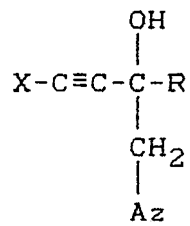

(IV)

in welcher
R und X die oben angegebene Bedeutung haben und
Az für 1,2,4-Triazol-1-yl oder Imidazol-1-yl steht.

Nach dem bisher bekannten Verfahren zur Herstellung von Verbindungen der Formel (IV) war es erforderlich, Acetylen-Derivate der Formel

X-C≡C-H (V)

in welcher
X die oben angegebene Bedeutung hat,
zunächst durch Umsetzung mit starken Basen, wie z.B. n-Butyl-lithium, zu metallieren und die dabei entstehenden metallorganischen Verbindungen dann mit Azolyl-ketonen der Formel

$Az-CH_2-CO-R$ (VI)

in welcher
R und Az die oben angegebene Bedeutung haben,
umzusetzen (vgl. EP-A 0 108 995). Mit Hilfe der erfindungsgemäß herstellbaren Acetylenepoxide ist es nun möglich, die Stoffe der Formel (IV) ohne Verwendung von metallorganischen Verbindungen herzustellen.

Verwendet man 1-Fluor-2,2-dimethyl-5-(4'-chlorphenyl)-4-pentin-3-on und Trimethylsulfonium-methylsulfat als Ausgangsstoffe und Kalium-tert.-butanolat als Base, so kann der Verlauf des erfindungsgemäßen Verfahrens durch das folgende Formelschema wiedergegeben werden:

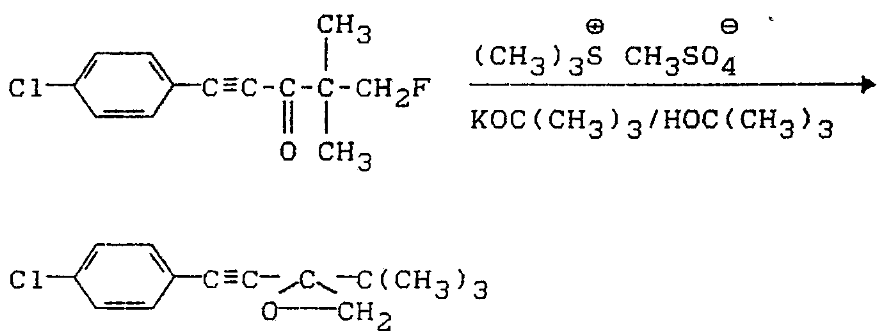


Die bei dem erfindungsgemäßen Verfahren als Ausgangsstoffe benötigten Acetylenketone sind durch die Formel (II) allgemein definiert. In dieser Formel stehen vorzugsweise

X für geradkettiges oder verzweigtes Alkyl mit 1 bis 4 Kohlenstoffatomen sowie für gegebenenfalls einfach bis fünffach, gleich oder verschieden substituiertes Phenyl, wobei als Substituenten genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, wie Fluor- oder Chloratomen, sowie jeweils gegebenenfalls durch Halogen und/oder Alkyl mit 1 bis 2 Kohlenstoffatomen substituiertes Phenyl, Phenoxy, Benzyl und Benzyloxy; und

R für die Gruppierungen

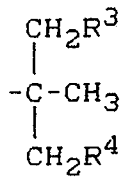 , 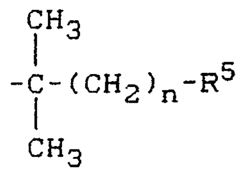 und 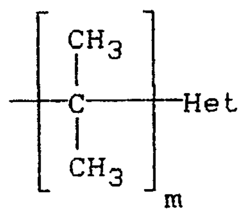

wobei

$R^3$ für Wasserstoff, Fluor, Chlor oder Brom steht,

$R^4$ für Fluor, Chlor oder Brom steht,

$R^5$ für Alkylthio mit 1 bis 4 Kohlenstoffatomen, sowie für jeweils gegebenenfalls einfach bis fünffach, gleich oder verschieden substituiertes Phenyl, Phenoxy und Phenylthio steht, wobei jeweils als Phenylsubstituenten vorzugsweise genannt seien: Halogen, Alkyl mit 1 bis 4 Kohlenstoffatomen; Alkoxy und Alkylthio mit jeweils 1 bis 2 Kohlenstoffatomen; Halogenalkyl, Halogenalkoxy und Halogenalkylthio mit jeweils 1 bis 2 Kohlenstoffatomen und 1 bis 5 gleichen oder verschiedenen Halogenatomen, wie insbesondere Fluor- und Chloratomen, sowie gegebenenfalls durch Halogen substituiertes Phenyl,

n für die Zahlen 0, 1 oder 2 steht,

Het für jeweils gegebenenfalls einfach bis vierfach, gleich oder verschieden substituiertes Dioxolan-2-yl oder 1,3-Dioxanyl steht, wobei als Substituenten genannt seien: Alkyl mit 1 bis 4 Kohlenstoffatomen sowie jeweils gegebenenfalls einfach bis fünffach, gleich oder verschieden substituiertes Phenyl und Phenoxyalkyl mit 1 bis 4 Kohlenstoffatomen im Alkylteil, wobei als Phenylsubstituenten genannt seien: Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen, wie vorzugsweise Fluor- und Chloratomen, und

m für die Zahlen 0 oder 1 steht.

Besonders bevorzugt sind diejenigen Verbindungen der Formel (II), in denen

X für gegebenenfalls einfach bis dreifach, gleich oder verschieden substituiertes Phenyl steht, wobei als Substituenten genannt seien: Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen; und

R für die Gruppierung

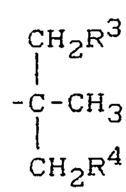 ,

steht,

wobei

$R^3$ für Wasserstoff oder Fluor steht und

$R^4$ für Fluor steht.

Eine weitere Gruppe besonders bevorzugter Stoffe sind diejenigen Verbindungen der Formel (II), in denen

X für Methyl, Ethyl, tert.-Butyl sowie für gegebenenfalls einfach bis dreifach, gleich oder verschieden substituiertes Phenyl steht, wobei als Substituenten genannt seien: Fluor, Chlor, Brom, Methyl, tert.-Butyl, Methoxy, Methylthio, Trifluormethyl, Trifluormethoxy, Trifluormethylthio sowie jeweils gegebenenfalls durch Fluor, Chlor und/oder Methyl substituiertes Phenyl, Phenoxy, Benzyl oder Benzyloxy; und

R für die Gruppierungen

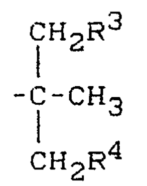 , 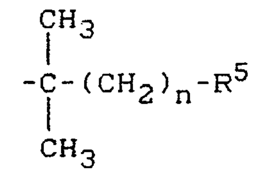 und 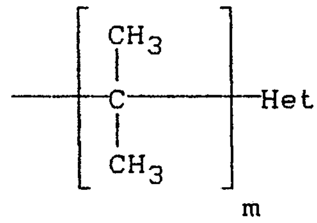

wobei

- $R^3$ für Wasserstoff, Fluor oder Chlor steht,
- $R^4$ für Fluor oder Chlor steht,
- $R^5$ für Methylthio, Ethylthio, sowie für jeweils gegebenenfalls einfach oder zweifach, gleich oder verschieden substituiertes Phenyl, Phenoxy, Phenylthio, steht, wobei jeweils als Phenylsubstituenten genannt seien: Fluor, Chlor, Methyl, Ethyl, Methoxy, Methylthio, Trifluormethyl, Trifluormethoxy und Trifluormethylthio;
- n für die Zahlen 0, 1 oder 2 steht;
- Het für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten genannt seien: Methyl, Ethyl, n-Propyl, Isopropyl sowie jeweils gegebenenfalls einfach bis dreifach, gleich oder verschieden durch Fluor, Chlor, Methyl, Trifluormethyl oder Trifluormethoxy substituiertes Phenyl und Phenoxymethyl und
- m für die Zahlen 0 oder 1 steht.

Die Acetylenketone der Formel (II) sind bekannt oder lassen sich nach prinzipiell bekannten Verfahren in einfacher Weise herstellen (vgl. EP-A 0 144 044).

Die bei dem erfindungsgemäßen Verfahren weiterhin als Ausgangsstoffe benötigten Trimethylsulfonium-Salze der Formel (III) sind ebenfalls bekannt (vgl. Heterocycles 8, 397 (1977); J. Amer, Chem. Soc, 84, 3782-3783 (1962); J. Amer. Chem. Soc. 87, 1353-1364 (1965) und Tetrahedron Letters 1962, 661). Die Trimethylsulfonium-Salze werden bei den erfindungsgemäßen Umsetzungen in frisch hergestelltem Zustand verwendet, indem man sie durch Reaktion von Dimethylsulfid mit Dimethylsulfat, Methylbromid oder Methyliodid in situ erzeugt.

Als Alkalimetall-tert.-butanolate kommen bei der Durchführung des erfindungsgemäßen Verfahrens vorzugsweise Kalium-tert.-butanolat oder Natrium-tert.-butanolat infrage.

Als Verdünnungsmittel dient bei der Durchführung des erfindungsgemäßen Verfahrens tert.-Butanol, gegebenenfalls im Gemisch mit Dimethylsulfid. Verwendet man Gemische aus tert.-Butanol und Dimethylsulfid, so kann der Anteil der Solventien innerhalb eines größeren Bereiches variiert werden. Im allgemeinen führt man die Herstellung des Trimethylsulfonium-Salzes in Gegenwart eines Überschusses von Dimethylsulfid durch. Danach wird tert.-Butanol zur Durchführung der folgenden Umsetzung hinzugefügt.

Die Reaktionstemperaturen können bei der Durchführung des erfindungsgemäßen Verfahrens innerhalb eines größeren Bereiches variiert werden. Im allgemeinen arbeitet man sowohl bei der Herstellung des Trimethylsulfoniumsalzes als auch bei dessen anschließender Umsetzung mit einem Acetylenketon der Formel (II) bei Temperaturen zwischen -20 °C und +60 °C, vorzugsweise zwischen 0 °C und 30 °C.

Das erfindungsgemäße Verfahren wird im allgemeinen bei Normaldruck durchgeführt. Es ist aber auch möglich, unter erhöhtem oder vermindertem Druck zu arbeiten.

Bei der Durchführung des erfindungsgemäßen Verfahrens werden die Mengen an den Reaktionskomponenten im allgemeinen so gewählt, daß auf 1 Mol an Acetylenketon der Formel (II) 1,0 bis 2,0 Mol, vorzugsweise 1,0 bis 1,5 Mol an Trimethylsulfonium-Salz der Formel (III) sowie 1,0 bis 2,0 Mol, vorzugsweise 1,0 bis 1,6 Mol, an Alkalimetall-tert.-butanolat eingesetzt werden.

Im einzelnen geht man bei der Durchführung des erfindungsgemäßen Verfahrens so vor, daß man Trimethylsulfonium-Salz vorlegt oder in der angegebenen Weise unmittelbar zuvor erzeugt, dann tert.-Butanol und Acetylenketon hinzufügt und danach Alkalimetall-tert.-butanolat, gegebenenfalls in Gegenwart von tert.-Butanol, zusetzt. Es ist aber auch möglich, das Trimethylsulfonium-Salz zu einer Mischung der übrigen Reaktionskomponenten hinzuzugeben. Die Aufarbeitung erfolgt nach üblichen Methoden. Im allgemeinen geht man so vor, daß man das Verdünnungsmittel abzieht, den verbleibenden Rückstand in Wasser aufnimmt, die Phasen trennt, dann die wäßrige Phase mehrfach mit einem in Wasser wenig löslichen organischen Solvens extrahiert, anschließend die vereinigten organischen Phasen wäscht und nach dem Trocknen einengt.

Die erfindungsgemäß herstellbaren Acetylenketone der Formel (I) sind neu, Sie sind wertvolle Zwischenprodukte zur Synthese von bekannten Hydroxyalkinyl-azolyl-Derivaten mit fungizider Wirksamkeit. So lassen sich erfindungsgemäß herstellbare Acetylenepoxide der Formel (I) mit Azolen der Formel

HAz (VII)

in welcher
Az für 1,2,4-Triazol-1-yl oder Imidazol-1-yl steht,
in Gegenwart eines Säurebindemittels, wie z.B. Kaliumcarbonat, und in Gegenwart eines inerten organischen Verdünnungsmittels, wie z.B. Acetonitril, Dimethylformamid oder N-Methyl-pyrrolidon bei Temperaturen zwischen 50 °C und 150 °C umsetzen, um Hydroxyalkinyl-azolyl-Derivate der Formel

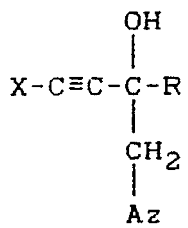

(IV)

in welcher
R, X und Az die oben angegebene Bedeutung haben,
zu erhalten.

Die erfindungsgemäße Herstellung von Acetylenepoxiden und deren Verwendung als Zwischenprodukte werden durch die folgenden Beispiele veranschaulicht.

Herstellungsbeispiele

Beispiel 1

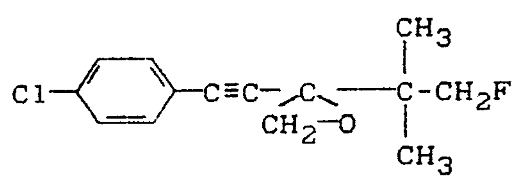

Bei 20 °C werden 207,5 g (156 ml = 1,65 Mol) Dimethylsulfat unter Rühren in 558 g (660 ml = 9 Mol) Dimethylsulfid gegeben, wobei die Temperatur des Gemisches durch exotherme Reaktion der Substanzen bis auf 38 °C ansteigt. Danach läßt man 750 ml tert.-Butanol zulaufen und fügt bei 20 °C unter Rühren 358 g (1,5 Mol) 1-Fluor-2,2-dimethyl-5-(4'-chlorphenyl)-4-pentin-3-on hinzu. Anschließend wird noch 30 Minuten bei 20 °C gerührt, wobei sich eine homogene Lösung bildet. Dann wird das Reaktionsgemisch auf 10 °C abgekühlt und innerhalb von 75 Minuten wird eine Lösung von 193 g (1,75 Mol) Kalium-tert.-butanolat in 1.350 ml tert.-Butanol zugetropft. Man rührt 3 Stunden bei 10 °C nach und zieht dann bei maximal 30 °C das Verdünnungsmittel unter vermindertem Druck ab. Der Rückstand wird in Wasser aufgenommen und die Phasen werden getrennt. Die wäßrige Phase wird dreimal mit Methylenchlorid extrahiert. Die vereinigten organischen Phasen werden mit Wasser gewaschen und nach dem Trocknen über Natriumsulfat unter vermindertem Druck eingeengt. Es verbleiben 336 g eines Öls, das zu 94 Gew.-% aus dem gewünschten Produkt besteht. Die Ausbeute an 2-(4'-Chlorphenyl-ethinyl)-2-(2'-fluor-1',1'-dimethyl)-ethyl-oxiran errechnet sich danach zu 83,6 % der Theorie.

In entsprechender Weise und nach den in der Beschreibung angegebenen Verfahrensbedingungen werden die in der folgenden Tabelle formelmäßig aufgeführten Verbindungen hergestellt.

Tabelle

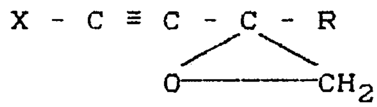 (I)

| Bsp. Nr. | X | R | Ausbeute in % der Theorie | Physikal. Konstante |
|---|---|---|---|---|
| 2 | 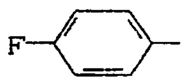 | $-C(CH_3)_2CH_2F$ | 78 | Öl |
| 3 | 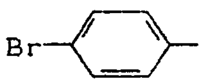 | " | 82 | " |
| 4 | 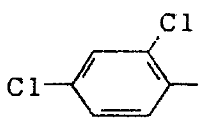 | " | 85 | " |
| 5 | 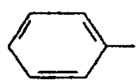 | " | 90 | " |
| 6 | 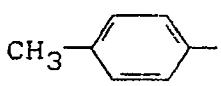 | " | 89 | " |
| 7 | 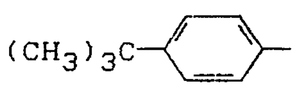 | " | 81 | " |

Verwendungsbeispiel

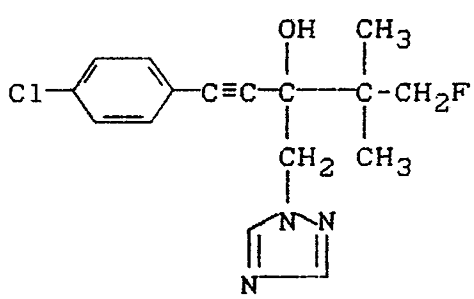

Zu einem Gemisch aus 3.500 ml Acetonitril, 146,65 g (2,125 Mol) 1,2,4-Triazol und 293,25 g gemahlenem Kaliumcarbonat werden bei Raumtemperatur unter Rühren 313,3 g (94 %ig = 1,169 Mol) 2-(4'-Chlorphenyl-ethinyl)-2-(2'-fluor-1',1'-dimethyl)-ethyl-oxiran zugetropft. Anschließend wird das Reaktionsgemisch 12 Stunden unter Rückfluß gekocht. Nach dem Abkühlen auf Raumtemperatur wird filtriert, der Rückstand mit Acetonitril gewaschen und das Filtrat unter vermindertem Druck eingeengt. Man suspendiert den dabei verbleibenden Rückstand in Wasser und erhitzt eine Stunde lang auf 60° C. Es wird heiß filtriert, der Rückstand wird getrocknet, dann mit n-Hexan aufgeschlämmt, einige Zeit gerührt und erneut abfiltriert. Es verbleiben 433 g eines Produktes, das in Essigester gelöst und über Kieselgel filtriert wird. Nach dem Abziehen des Lösungsmittels unter vermindertem Druck verbleiben 228,2 g eines Produktes, das zu 99 % aus 1-(4-Chlorphenyl)-4,4-dimethyl-5-fluor-3-(1,2,4-triazol-1-yl-methyl)-1-pentin-3-ol besteht. Die Ausbeute erreichnet sich danach zu 60 % der Theorie.
Schmelzpunkt: 120° C.

Vergleichsbeispiel A

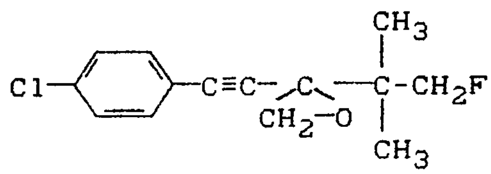

In ein Gemisch aus 20,4 g (0,1 Mol) Trimethylsulfoniumiodid und 60 ml absolutem Acetonitril wird bei Raumtemperatur unter Rühren eine Lösung von 9,54 g (0,05 Mol) 1-Fluor-2,2-dimethyl-5-(4'-chlorphenyl)-4-pentin-3-on in 30 ml absolutem Acetonitril eingetropft. Anschließend werden bei Temperaturen zwischen 10 und 20° C 5,4 g (0,1 Mol) Natriummethylat portionsweise zugegeben. Man rührt 4 Stunden bei Raumtemperatur nach, saugt ab, wäscht mit Acetonitril nach und engt das Filtrat unter vermindertem Druck ein. Der verbleibende Rückstand wird in Methylenchlorid aufgenommen, die entstehende Lösung wird mit Wasser gewaschen und erneut eingeengt. Man erhält 3,75 g eines Öls, das gemäß Gaschromatogramm zu 10,9 % aus 2-(4'-Chlorphenyl-ethinyl)-2-(2'-fluor-1',1'-dimethyl)-ethyl-oxiran besteht. Die Ausbeute errechnet sich danach zu 3,2 % der Theorie.

Vergleichsbeispiel B

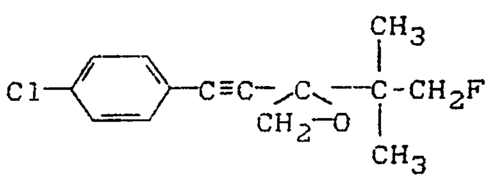

Ein Gemisch aus 10,8 ml (0,15 Mol) Dimethylsulfid, 12 ml (0,127 Mol) Dimethylsulfat und 10 ml absolutem Tetrahydrofuran wird 16 Stunden bei 20° C gerührt. Anschließend werden zunächst 150 ml absolutes Tetrahydrofuran und dann 23,8 g (0,1 Mol) 1-Fluor-2,2-dimethyl-5-(4'-chlorphenyl)-4-pentin-3-on in 40 ml Toluol zugetropft. Dann werden bei 10° C 20,6 g (0,184 Mol) Kalium-tert.-butanolat zugegeben. Man rührt 4 Stunden bei 20° C nach, entfernt dann die Hauptmenge des Lösungsmittels unter vermindertem Druck bei Temperaturen unterhalb von 30° C, fügt dann 120 ml wäßrige Natriumhypochlorit-Lösung hinzu und extrahiert die wäßrige Phase dreimal mit Toluol. Die vereinigten organischen Phasen werden mit Wasser gewaschen und unter vermindertem Druck eingeengt. Es verbleiben 20,6 g eines Öls, das zu 27,3 % aus 2-(4'-Chlorphenyl-ethinyl)-2-(2'-fluor-1',1'-dimethyl)-ethyl-oxiran besteht. Die Ausbeute errechnet sich danach zu 22,2 % der Theorie.

**Patentansprüche**

**1.** Verfahren zur Herstellung von Acetylenepoxiden der Formel

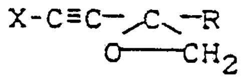

(I)

in welcher

X für gegebenenfalls substituiertes Phenyl oder Alkyl steht und
R für die Gruppierungen

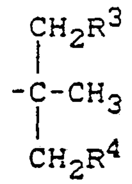 , 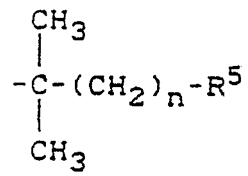 und 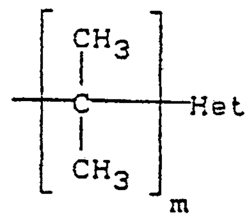

steht, wobei
$R^3$ für Wasserstoff oder Halogen steht,
$R^4$ für Halogen steht,
$R^5$ für Alkylthio sowie für jeweils gegebenenfalls substituiertes Phenyl, Phenoxy und Phenylthio steht,
n für die Zahlen 0, 1 oder 2 steht,
m für die Zahlen 0 oder 1 steht und
Het für gegebenenfalls substituiertes Dioxolanyl oder Dioxanyl steht,

dadurch gekennzeichnet, daß man Acetylenketone der Formel

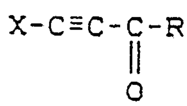

(II)

in welcher

R und X die oben angegebene Bedeutung haben,

mit Trimethylsulfoniumsalzen der Formel

$$(CH_3)_3\overset{\oplus}{S}\ \overset{\ominus}{Z} \qquad (III)$$

in welcher

Z für Brom, Iod oder Methylsulfat steht,

in Gegenwart von Alkalimetall-tert.-butanolat und in Gegenwart von tert.-Butanol und gegebenenfalls Dimethylsulfid umsetzt.

**2.** Verfahren gemäß Anspruch 1 zur Herstellung von Acetylenepoxid der Formel

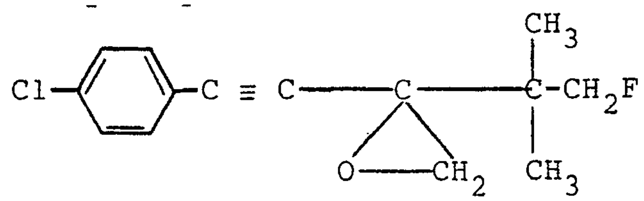

,

dadurch gekennzeichnet, daß man 1-Fluor-2,2-dimethyl-5-(4'-chlorphenyl)-4-pentin-3-on als Acetylenketon der Formel (II) und Trimethylsulfonium-methylsulfat als Trimethylsulfoniumsalz der Formel (III) einsetzt.

3. Verfahren gemäß Anspruch 1, dadurch gekennzeichnet, daß man erhaltene Acetylenepoxide der Formel (I) mit Azolen der Formel

HAz (VII)

in welcher

Az für 1,2,4-Triazol-1-yl oder Imidazol-1-yl steht,

in Gegenwart eines Säurebindemittels und in Gegenwart eines inerten organischen Verdünnungsmittels umsetzt, um Hydroxyalkinyl-azolyl-Derivate der Formel

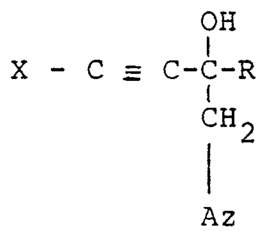

in welcher
R, X und Az die oben angegebene Bedeutung haben,
zu erhalten.

## Claims

1. Process for the preparation of acetylene epoxides of the formula

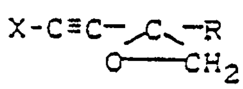

(I)

in which

X represents optionally substituted phenyl or alkyl and

R represents the groups

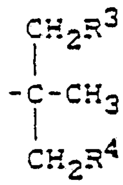 , 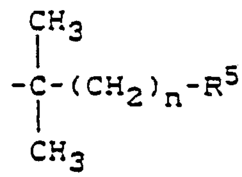 and 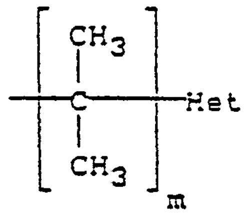

in which

| | |
|---|---|
| $R^3$ | represents hydrogen or halogen, |
| $R^4$ | represents halogen, |
| $R^5$ | represents alkylthio and in each case optionally substituted phenyl, phenoxy and phenylthio, |
| n | represents the numbers 0, 1 or 2, |
| m | represents the numbers 0 or 1 and |
| Het | represents optionally substituted dioxolanyl or dioxanyl, |

characterised in that acetylene ketones of the formula

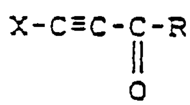 (II)

in which

R and X have the abovementioned meaning,

are reacted with trimethylsulphonium salts of the formula

$$(CH_3)_3\overset{\oplus}{S}\ \overset{\ominus}{Z} \quad (III)$$

in which

Z represents bromine, iodine or methyl sulphate,

in the presence of an alkali metal tert.-butanolate and in the presence of tert.-butanol and optionally dimethyl sulphide.

**2.** Process according to Claim 1 for the preparation of acetylene epoxide of the formula

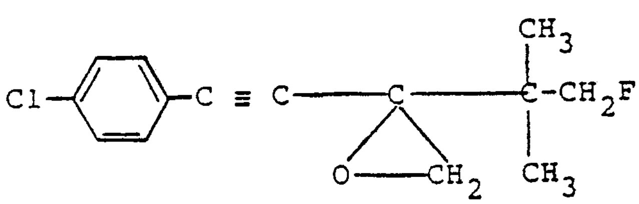 ,

characterised in that 1-fluoro-2,2-dimethyl-5-(4'-chlorophenyl)-4-pentin-3-one is used as acetylene ketone of the formula (II) and trimethylsulphoniummethyl sulphate is used as trimethylsulphonium salt of the formula (III).

**3.** Process according to Claim 1, characterised in that the resultant acetylene epoxides of the formula (I)

are reacted with azoles of the formula

HAz (VII)

in which

Az represents 1,2,4-triazol-1-yl or imidazol-1-yl,

in the presence of an acid acceptor and in the presence of an inert organic diluent, in order to obtain hydroxyalkinyl-azolyl derivatives of the formula

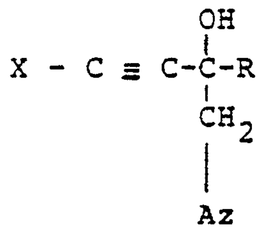

in which

R, X and Az have the abovementioned meaning.

**Revendications**

**1.** Procédé pour la préparation d'époxydes d'acétylène de formule

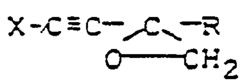

(I)

dans laquelle

X représente un groupe alkyle ou un groupe phényle, éventuellement substitué et
R représente les groupements

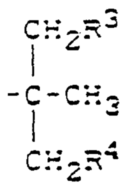

,

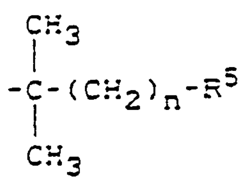

und

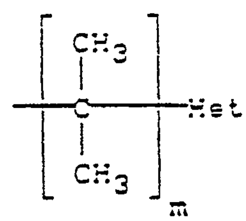

où
$R^3$ représente un atome d'hydrogène ou un atome d'halogène,
$R^4$ représente un atome d'halogène,
$R^5$ représente un groupe alkylthio, ainsi qu'un groupe phényle, un groupe phénoxy et un groupe phénylthio, chacun étant éventuellement substitué,
n représente les chiffres 0, 1 ou 2,
m représente les chiffres 0 ou 1 et
Het représente un groupe dioxolanyle ou un groupe dioxanyle, éventuellement substitué,

**caractérisé en ce qu**'on fait réagir des acétylène-cétones de formule

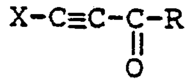 (II)

dans laquelle

R et X ont la signification mentionnée ci-dessus,

avec des sels de triméthylsulfonium de formule

$$(CH_3)_3S^{\oplus} Z^{\ominus} \quad (III)$$

où

Z représente un atome de brome, un atome d'iode ou un groupe méthylsulfate,

en présence d'un butanolate tertiaire d'un métal alcalin et en présence d'un butanol tertiaire, et éventuellement de diméthylsulfure.

**2.** Procédé selon la revendication 1 pour la préparation d'époxyde d'acétylène de formule

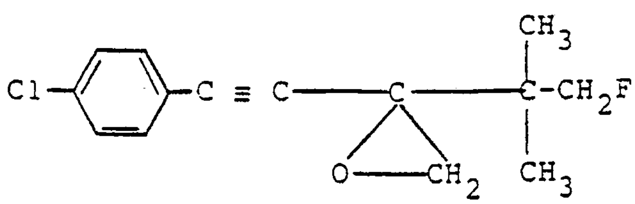

**caractérisé en ce qu**'on met en oeuvre la 1-fluoro-2,2-diméthyl-5-(4'-chlorophényl)-4-pentyn-3-one comme acétylène-cétone de formule (II) et le méthylsulfate de triméthylsulfonium comme sel de triméthylsulfonium de formule (III).

**3.** Procédé selon la revendication 1, **caractérisé en ce qu**'on fait réagir les époxydes d'acétylène de formule (I) que l'on a obtenus, avec des azoles de formule

HAz (VII)

dans laquelle

Az représente un groupe 1,2,4-triazol-1-yle ou un groupe imidazol-1-yle,

en présence d'un agent liant les acides et en présence d'un diluant organique inerte, pour obtenir des dérivés d'hydroxyalcynyl-azolyle de formule

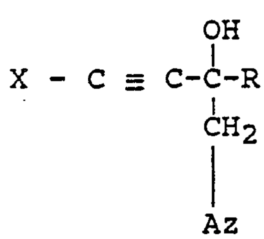

dans laquelle

R, X et Az ont la signification mentionnée ci-dessus.